Europäisches Patentamt

European Patent Office

Office européen des brevets

(11) Veröffentlichungsnummer: **0 402 888**

**A1**

## (12) EUROPÄISCHE PATENTANMELDUNG

(21) Anmeldenummer: **90111174.0**

(22) Anmeldetag: **13.06.90**

(51) Int. Cl.5: **G01N 33/53, B01L 3/00, C12M 1/22**

(30) Priorität: **16.06.89 DE 3919690**

(43) Veröffentlichungstag der Anmeldung:
**19.12.90 Patentblatt 90/51**

(84) Benannte Vertragsstaaten:
**AT BE CH DE FR GB IT LI LU NL SE**

(71) Anmelder: **BEHRINGWERKE**
**Aktiengesellschaft**
**Postfach 1140**
**D-3550 Marburg 1(DE)**

(72) Erfinder: **Fibi, Mathias, Dr.**
**Gabelsberger Strasse 11**
**D-3550 Marburg(DE)**
Erfinder: **Weyrich, Ludwig**
**Sonnenhang 14**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Inkubationsgefäss.**

(57) Bei dem zweiteiligen Inkubationsgefäß bestehend aus napfartigem Oberteil und gleichartigem Unterteil, die jeweils mit einem Flansch versehen sind, und deren Bodenteile mit den Seitenwänden des Unterteils den Inkubationsraum einschließen, sind die Seitenwände (2,2′,3,3′) von Oberteil (14) und Unterteil (15) gegen die Gefäßachse geneigt. Das Bodenteil (6) des Unterteils (15) ist mit einem Sprung (8) versehen, auf den sich das Bodenteil (7) des Oberteils (14) abstützt.

*Fig. 1*

EP 0 402 888 A1

## Inkubationsgefäß

Gegenstand der Erfindung ist ein zweiteiliges Inkubationsgefäß bestehend aus napfartigem Oberteil und gleichartigem Unterteil, die jeweils mit einem Flansch versehen sind und deren Bodenteile mit den Seitenwänden des Qnterteils einen Inkubationsraum einschließen.

Inkubationsgefäße der genannten Art dienen der Inkubation von Proben, beispielsweise Proteine oder Nukleinsäuren, die auf Filterpapier als Trägermaterial aufgebracht werden und mit einer zweiten Substanz, beispielsweise einem Protein oder einer Nukleinsäure, die beispielsweise radioaktiv, enzym- oder auf andere Weise markiert ist, inkubiert werden, um spezifische Wechselwirkungen mit der auf dem Filterpapier immobilisierten Probe nachzuweisen.

In der modernen biomedizinischen Forschung und Diagnostik haben sich molekularbiologische und immunologische Nachweismethoden durchgesetzt, weil mit ihnen hohe Spezifitäten und Sensivitäten erreicht werden können. So gehören beispielsweise alle Blotting-Methoden (Southern Blot, Northern Blot, Western Blot, Dot Blot, Slot Blot usw.) zu den Standardmethoden molekularbiologischer, immunologischer oder biochemischer Laboratorien. Alle Blotting-Techniken haben prinzipiell die Immobilisierung der zu untersuchenden Proben auf Filterpapieren gemeinsam. Beim Southern Blot, Northern Blot und Western Blot und Varianten davon werden die Proben vor der Zmmobilisierung beispielsweise in Agarose- oder Polyacrylamidgelen aufgetrennt. Bei den Dot Blot- oder Slot Blot-Verfahren werden die Proben mit Bilfe von Vakuum direkt auf die Filter aufgebracht. Die immobilisierten Proben werden dann direkt oder indirekt mit markierten Proben, beispielsweise Nukleinsäuren- oder Antikörperlösungen inkubiert und anschließend gewaschen. Die zurückbleibenden radioaktiven Signale werden auf Röntgenfilmen, zurückbleibende Enzymsignale durch Substratumwandlungen direkt auf dem Filter nachgewiesen.

Nach einer bekannten Methode werden die Filter zur Inkubation mit den Proben in Plastikfolien eingeschweißt. Das hat jedoch den Nachteil, daß die verwendeten Plastikfolien labil sind und daß es deshalb schwierig ist, das Filter luftblasenfrei zu versiegeln. Saüberes Einschweißen kann deshalb nur unter erheblichem Zeitaufwand erreicht werden. Außerdem entsteht bei radioaktivem Arbeiten mit dieser Methode viel radioaktiver Abfall durch radioaktive Kontamination von Schutzhandschuhen. Um Kontaminationen nicht zu verschleppen, müssen die Schutzhandschuhe häufig gewechselt werden. Zusätzlich entsteht viel radioaktiver Papierabfall, da die Folien nach dem Zuschneiden an der Schweißnaht gründlich gereinigt werden müssen. Auch bei vorsichtigem Arbeiten wird häufig die Schutzpapierunterlage auf dem Labortisch kontaminiert, so daß diese ebenfalls entsorgt werden muß. Auch Schweißgerät und Schere werden beim Zuschweißen bzw. Aufschneiden der Folien kontaminiert und müssen mit erheblichem Zeitaufwand gereinigt werden. Das durch diese Methode hervorgerufene, verhältnismäßig zeitintensive Eantieren mit radioaktiven Substanzen führt mitunter zu erheblicher Strahlenbelastung.

Bei einer anderen Inkubationsmethode werden Gefäße mit Deckel verwendet, wobei zwischen Inkubationsflüssigkeit und Deckel ein großer Luftraum vorhanden ist (EP-A-0 290 978). In diesem Gefäß verdunstet bei länger Inkubationszeit Wasser und sammelt sich am Deckel. Dadurch verändern sich die Pufferbedingungen, was mit exaktem Experimentieren nicht vereinbar ist. Außerdem ist es bei dieser Methode wegen der Verdunstung notwendig, mehr Inkubationsflüssigkeit einzusetzen, um ein Austrocknen des Filterpapiers zu vermeiden. So muß bei radioaktivem Arbeiten beispielsweise mehr Radioaktivität eingesetzt werden. Nach der Inkubation müssen die verwendeten Gefäße entweder mit Dekontaminationsflüssigkeit gründlich gereinigt werden, was erhebliche Zeit in Anspruch nimmt und die Strahlenbelastung erhöht, oder sie müssen entsorgt werden. Die verwendeten Gefäße sind meist nicht als Einwegartikel konzipiert und bestehen aus teurem, dickwandigen Material. Dadurch hat man hier neben dem Saübern der Gefäße hohe Materialkosten. Darüberhinaus besteht die Gefahr, daß bei mehrmaligem Verwenden der Inkubationsgefäße Meßergebnisse durch Spuren von Verunreinigungen verfälscht werden.

Hier will die Erfindung Abhilfe schaffen.

Die Erfindung löst die Aufgabe dadurch, daß die Seitenwände von Oberteil und Unterteil gegen die Gefäßachse geneigt sind und das Bodenteil des Unterteils mit einem Sprung versehen ist, auf dem sich das Bodenteil des Oberteils abstützt.

Die Neigung der Seitenwände gegen die Gefäßachse kann 3 bis 10, vorzugsweise 6° betragen. Mindestens zwei gegenüberliegende Seitenwände von Ober- und Unterteil können mit Sicken versehen sein, die ineinander zu liegen kommen, sobald sich das Bodenteil des Oberteils auf dem Sprung abstützt. Das Bodenteil des Oberteils kann ebenfalls einen Sprung aufweisen.

Das erfindungsgemäße Inkubationsgefäß ist zur Aufnahme aller Blottingpapiere sowie zur Inkubation von Proben hervorragend geeignet. Da es aus durchsichtigem Material hergestellt werden kann, ist der Blot in Sekundenschnelle bei direkter Kontrolle luftblasenfrei verpackt. Die Proben können über längere Zeit bei höheren Temperaturen inkubiert werden, ohne daß Nachteile durch Verdunsten oder Veränderung des Gefäßmaterials entstehen. Selbst das unversiegelte Gefäß zeigt bei längerer Inkubationszeit keine Flüssigkeitsverluste durch Verdunsten, da durch die Neigung der Wände gegen die Gefäßachse diese dampfdicht aufeinander zu liegen kommen. Das Inkubationsgefäß kann luft- und wasserdicht versiegelt und wieder geöffnet werden und erlaubt deshalb problemloses Pufferwechseln nach verschiedenen Inkubationsschritten. Mehrere gleichartige Inkubationsgefäße können gestapelt werden, so daß auf kleinstem Raum viele Blots gleichzeitig inkubiert werden können. Außerdem kann das Inkubationsgefäß an Filterformen und Größen, wie sie für Gelapparaturen, Dot Blot- und Slot Blot-Apparaturen verschiedener Fabrikate Verwendung finden, sowie an Rundfilter angepaßt werden, wodurch eine breite Anwendung möglich ist. Die Konstruktion des Inkubationsgefäßes ermöglicht die Verwendung von kostengünstigem Material, so daß Einwegverwendung vertretbar ist, wodurch Verfälschungen von Ergebnissen durch Verunreinigungen ausgeschlossen werden können.

Im folgenden wird die Erfindung anhand von lediglich einem Ausführungsweg darstellenden Zeichnungen näher erläutert. Es zeigt

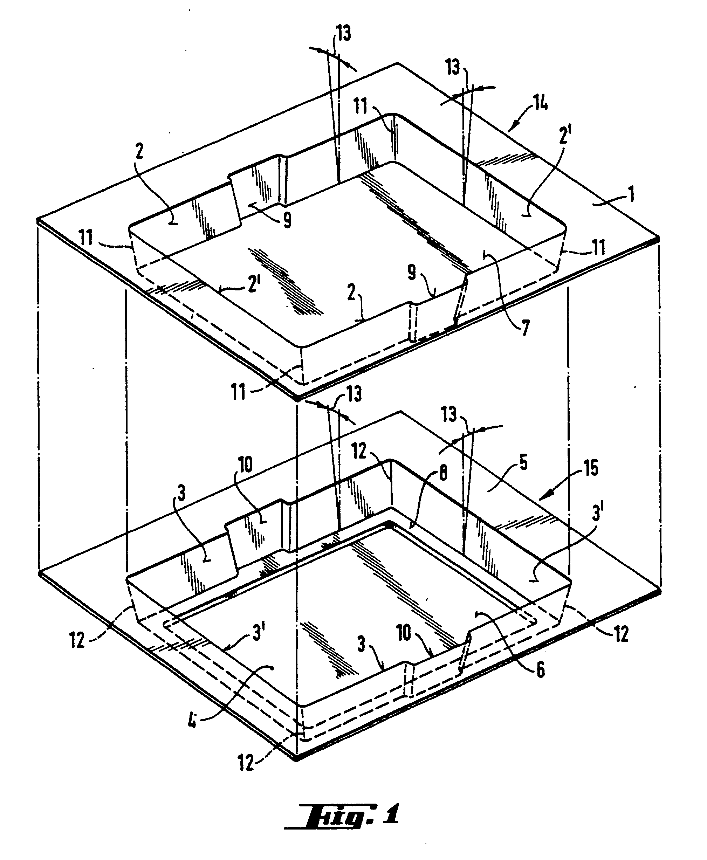

Figur 1 das Inkubationsgefäß perspektivisch und explodiert und

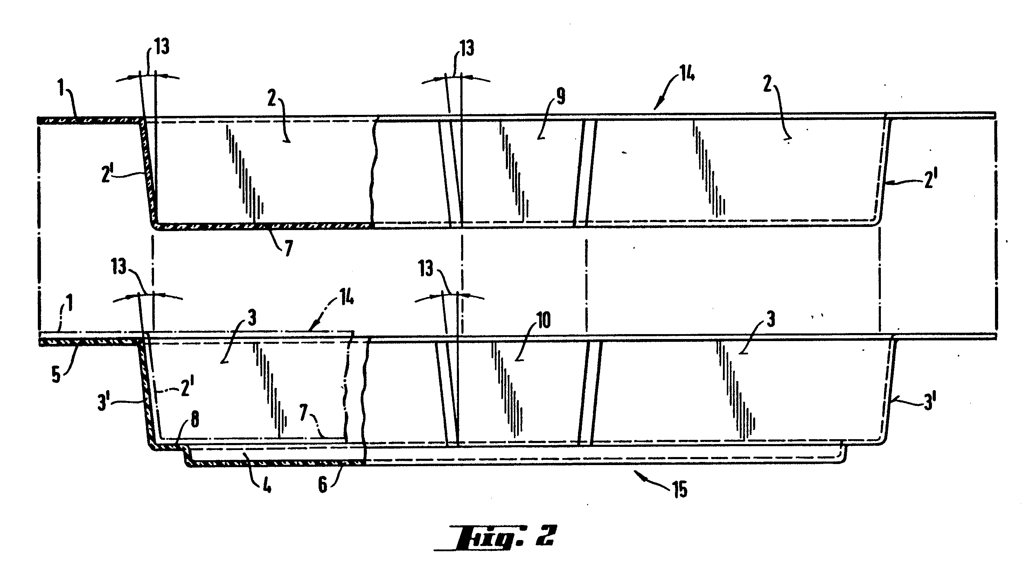

Figur 2 eine Seitenansicht explodiert.

Das zweiteilige Inkubationsgefäß besteht aus einem napfartigen Unterteil (15) und einem gleichartigen Oberteil (14). Oberteil (14) ist mit einem Flansch (1) und Unterteil (15) mit einem Flansch (5) versehen. Das Bodenteil (6) des Unterteils (15) weist einen Sprung (8) auf, auf dem sich das Bodenteil (7) des Oberteils (14) abstützt. Durch den Sprung (8) im Bodenteil (6) entsteht zwischen Bodenteil (6) und Bodenteil (7) der Inkubationoraum (4), in dem das Filter beschädigungsfrei abgelegt werden kann. Selbstverständlich kann auch das Bodenteil (7) des Oberteils (14) mit einem Sprung versehen sein, mit dem sich das Oberteil auf dem Sprung (8) des Qnterteils (15) abstützt (nicht dargestellt). Der Znkubationsraum (4) kann den Filterformen rund, quadratisch, rechteckig, etc. angepaßt werden. Alle Ecken (11,12) von Ober- und Unterteil (14,15) können aus Stabilitätsgründen abgerundet oder gefast sein. Zur Versteifung können die Seitenwände (2,3) mit Sicken (9,10) oder dergleichen versehen sein. Das Bodenteil (6) des Unterteils (15) ist plan und riefenfrei ausgebildet. Die Seitenwände (2,2',3,3') des Ober-und Unterteils (14,15) verlaufen schräg in Bezug auf die Gefäßachse, vorzugsweise unter einem Winkel von 6°, so daß der Gefäßquerschnitt vom Flansch (1,5) zum Bodenteil (6,7) hin abnimmt. Dadurch wird eine einwandfreie Eandhabung des Unterteils mit dem Oberteil gewährleistet. Da das Oberteil (14) mit Ausnahme der Inkubationswanne dem Unterteil (15) angepaßt ist, saugen sich die Seitenwände (2,2') des Oberteils an die Seitenwände (3,3') des Unterteils an. Der Werkstoff des Oberteils (14) kann der gleiche wie der des Unterteils (15) sein; bevorzugt werden transparente oder transluzente Werkstoffe eingesetzt. Allgemein können zur Eerstellung thermoplastische Folien aus Polyolefinen wie PE, PP, PVC oder Polystyrol verwendet werden. Als weitere Werkstoffe bieten sich steife Duroplaste oder andere Werkstoffe an.

Um ein Blottingpapier zu inkubieren, wird zunächst eine optimierte Menge Inkubationsflüssigkeit an einer Seite innerhalb der Inkubationswanne (4) verteilt. Dann wird das Filter mit der passenden Kante in die Flüssigkeit getaucht und in Richtung der gegenüberliegenden Seite so in den Inkubationsraum (4) gelegt, daß sich ein Teil der Flüssigkeit blasenfrei an der Unterseite des Filters verteilt. Ist dies erfolgt, so wird das Oberteil (14) an der gleichen Stelle schräg in die Flüssigkeit im Unterteil bis zum Sprung (8) eingetaucht. Die Inkubationsflüssigkeit wird dann durch vollständiges Rerunterdrücken des Oberteils auf der gegenüberliegenden Seite blasenfrei zwischen Oberseite des Filters und Bodenteil (7) des Oberteils (14) verteilt, und das Filter ist nach vollkommenen Entweichen der Luft blasenfrei verpackt. Oberteil (14) und Unterteil (15) können nun an den Flanschen (1,5) z.B. im Wärmeimpulsverfahren versiegelt werden. Es ist jedoch möglich, den Gefä3inhalt auch ohne Versiegelung über längere Zeit, beispielsweise über Nacht bei höheren Temperaturen von 70° C ohne Flüssigkeitsverlust zu inkubieren. Nach der Inkubation kann im Bedarfsfall die Versiegelungsnaht aufgeschnitten werden, das Oberteil kann vom Unterteil entfernt werden, und das Blottingpapier kann zum Waschen herausgenommen oder im Unterteil gewaschen werden. Durch die wasserabstoßenden Eigenschaften des Gefäßmaterials können die Flüssigkeiten sehr leicht aus dem Gefäß entfernt werden.

**Ansprüche**

1. Zweiteiliges Inkubationsgefäß bestehend aus napfartigem Oberteil und gleichartigem Unterteil, die jeweils mit einem Flansch versehen sind, und deren Bodenteile mit den Seitenwänden des Unterteils den Inkubationsraum einschließen, dadurch gekennzeichnet, daß die Seitenwände (2,2',3,3') von Oberteil (14) und Unterteil (15) gegen die Gefäßachse geneigt sind und das Bodenteil (6) des Unterteils (15) mit einem Sprung (8) versehen ist, auf dem sich das Bodenteil (7) des Oberteils (14) abstützt.

2. Zweiteiliges Inkubationsgefäß nach Anspruch 1, dadurch gekennzeichnet, daß die Seitenwände (2,2´,3,3´) von Oberteil (14) und Unterteil (15) um 3 bis 10°, vorzugsweise um 6° gegen die Gefäßachse geneigt sind.

3. Zweiteiliges Inkubationsgefäß nach Anspruch 1 oder 2, dadurch gekennzeichnet, daß mindestens zwei Seitenwände (2,3) von Ober- und Unterteil (14,15) mit Sicken (9,10) versehen sind, die ineinandergreifen, sobald sich das Bodenteil (7) des Oberteils (14) auf dem Sprung (8) abstützt.

4. Zweiteiliges Inkubationsgefäß nach einem der Ansprüche 1 bis 3, dadurch gekennzeichnet, daß das Bodenteil (7) des Oberteils (14) einen Sprung aufweist, mit dem sich das Oberteil (14) auf dem Sprung (8) des Qnterteils (15) abstützt.

**Fig. 1**

*Fig. 2*

EP 0 402 888 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-4321330 (BAKER ET AL.) <br> * Zusammenfassung; Ansprüche ; Figuren 1-3 * <br> --- | 1-4 | G01N33/53 <br> B01L3/00 <br> C12M1/22 |
| Y | US-A-4795562 (WALSH) <br> * Ansprüche 1-3, 5; Figuren 1, 3, 5 * <br> --- | 1-4 | |
| Y,D | EP-A-290978 (LIFE TECHNOLOGIES INC.) <br> * Spalte 3, Zeile 41 - Spalte 6, Zeile 5; Ansprüche 1-10; Figuren * <br> --- | 1-4 | |
| A | EP-A-245994 (ICI AUSTRALIA LIMITED) <br> * Zusammenfassung; Figur 3 * <br> ----- | 1-4 | |
| | | | **RECHERCHIERTE SACHGEBIETE (Int. Cl.5 )** <br><br> C12M <br> G01N <br> B01L <br> B65D |

Der vorliegende Recherchenbericht wurde für alle Patentansprüche erstellt

| Recherchenort | Abschlußdatum der Recherche | Prüfer |
|---|---|---|
| DEN HAAG | 01 OKTOBER 1990 | EPAILLARD P.J.H. |

EPO FORM 1503 03.82 (P0403)